# EUROPEAN PATENT APPLICATION

(11) **EP 1 683 547 A1**
(43) Date of publication of application: **26.07.2006**
(21) Application number: 06425028.5
(22) Date of filing: 20.01.2006
(51) Int. Cl.: A61Q 11/00, A61K 8/97

(54) **Anticaries toothpaste containing Rubus Fruticosus for treating and cleaning teeth and/or gums**

(30) Priority: 21.01.2005 IT MI20050068
(71) Applicant: Abbruzzo, Ignazio, 20142 Milano (IT)
(72) Inventor: Abbruzzo, Ignazio, 20142 Milano (IT)
(74) Representative: Sarpi, Maurizio

(57) **Abstract**

The present invention discloses a toothpaste containing Rubus Fruticosus as active principle able to fight dental caries; said toothpaste is also capable of treating gums and cleaning teeth.

## Description

The present invention relates to anticaries toothpaste having as active principle Rubus Fruticosus capable of providing for the health of gums and cleaning of teeth.

It is known that a bad dental care can cause infections, periodontal illness, and dental caries. If teeth are not brushed, a soft mass at the surface of the teeth, the so-called "dental plaque", quickly develops right after one or two days.

The "dental plaque" develops in different stages, the first one consisting of the deposit of salivary mucoids onto the surface of the teeth which are coated and deeply impregnated by the bacteria of the oral cavity. These bacteria reproduce continuously and, according to their species, can secrete by-products which are harmful for teeth and gums.

A good source of nutritive principles for the bacterial reproduction consists of the particles of food remaining in the mouth.

During the last stages of formation of the "dental plaque", the deposit becomes pregnant with a remarkable amount of hydroxy apatite which sets little by little, thus forming tartar which is usually removed only by the dentist

A number of attempts were made to check the formation of "dental plaque"; for example, mixtures of enzymes that can be administered for oral use have been included in classic sanitary products. These products contain several enzymes such as protease, lipase, and amylase to break and disintegrate the "dental plaque".

However, some mixtures are obtained from animals or plants and are not available in sufficient amounts and sometimes difficult to find because of the large amount requested for the dental products. Accordingly, the cost of such mixtures is high because of the limited sources of supply. Further mixtures available from microorganisms have components that can be mutually deactivated, thus reducing the activity of the enzymes.

In the same way, some mixtures have components with extreme values of pH to provide an optimum activity, thus reducing their effectiveness in the mouth.

The Applicant has now surprisingly found a toothpaste containing Rubus Fruticosus which has the property of fighting dental caries.

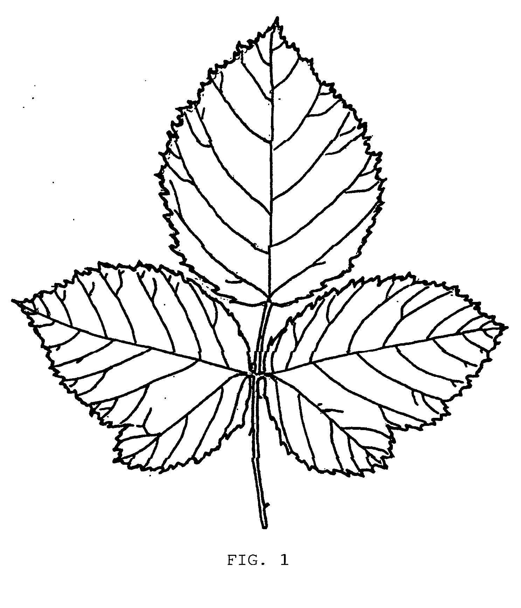

Rubus Fruticosus or bramble is a low-cost, widely spread, easily available plant. Rubus Fruticosus is a spiny plant showing infructescences of the blackberry type. It has very prickly, thriving branches, leaves with a whitish, lower face (see Fig. 1), and white-rosy flowers. The infructescence has a sweet and sour taste and is used to prepare jam and refreshing drinks.

Rubus Fruticosus is a widely known plant also mentioned in the book of Exodus as JAHVE manifests himself to Moses the first time through a bramble bush on fire (burning Bramble Bush).

Folk medicine has been using its buds for a long time because of their astringent, refreshing features. A variety of species of the Rubus genus such as THYRSANTHUS, UMBRIFOLIUS, TRIVIALIS, HTSPIDUS, LACINIATUS, PRACUDENS, VITIFOLIUS and LINGIFOLIUS are known; all of these variety have the same features as FRUTICOSUS.

The object of the present invention is then an anticaries toothpaste containing Rubus Fruticosus for treating and cleaning teeth and/or gums. Such toothpaste proves effective for the teeth and/or gum care as well as for the prevention of caries; its active principle (Rubus Fruticosus) is easily available also in large amounts and at low cost.

In particular, Rubus Fruticosus is present in a minimum amount of 10% until a maximum amount of 20%-wt/wt in the toothpaste of the present invention.

Such toothpaste includes the basic ingredients which are known to those skilled in the art for the formulation of dentifrices such as, for example, bleaching agents, abrasive agents, viscous agents, surface-active agents, solvents, sweeteners, flavouring agents.

Another object of the present invention is a method for preparing a toothpaste with anticaries activity consisting of:
- cold macerating 100 g of Rubus Fruticosus in 10 centilitres of a suitable solvent for about 5 days as a maximum;
- mixing, according to known techniques, the previously prepared macerated product with the basic ingredients of a toothpaste such as, for example, purified water, sorbitol, glycerine, sodium bicarbonate, natural fragrances, and liquorice.

The solvent used for macerating Rubus Fruticosus is in particular purified water but other suitable solvents such as essential oils and alcohol can be used.

Another object of the present invention consists of the use of Rubus Fruticosus for the preparation of a composition for oral use for the treatment and prevention of caries.

A not limiting example of preparation of an anticaries toothpaste containing Rubus Fruticosus is provided below.

### Example 1

The anticaries toothpaste includes 20%-wt/wt of Rubus Fruticosus as well as the following ingredients:
- bleaching agents 34%
- wetting agents 20%
- viscous agents 2%
- binding agents
- surface-active agents 2%
- water 20%
- sweeteners 0.5%
- flavouring agents 0.5%.

## Claims

1. An anticaries toothpaste containing Rubus Fruticosus for treating and cleaning teeth and/o gums.

2. The toothpaste according to claim 1, wherein the amount of Rubus Fruticosus is about 10% to 20%-wt/wt.

3. The toothpaste according to claims 1 and 2, wherein the amount of Rubus Fruticosus is about 20%-wt/wt in a mixture with purified water, sorbitol, glycerine, sodium bicarbonate, natural fragrances, and liquorice.

4. A method of preparing an anticaries toothpaste containing Rubus Fruticosus, **characterized in that**:
- cold macerating 100 g of leaves of Rubus Fruticosus in 10 centilitres of a suitable solvent selected among purified water, essential oils or alcohols for about 5 days;
- mixing the macerated product with the basic ingredients of a toothpaste.

5. The method according to claim 4, wherein the solvent is purified water.

6. Use of Rubus Fruticosus for preparing anticaries compositions for oral use.
